# EUROPEAN PATENT SPECIFICATION

(11) **EP 0 908 460 B1**
(45) Date of publication and mention of the grant of the patent: **15.01.2003**
(21) Application number: 97402349.1
(22) Date of filing: 06.10.1997
(51) Int. Cl.: C07D 493/20, C11B 9/02, A61K 31/365

(54) **A process for the simultaneous production of artemisinin and essential oil from the plant artemisia annua**
Verfahren zur gleichzeitigen Produktion von Artemisinin und ätherisches Öl aus der Pflanze Artemisia Annua
Procédé pour la production simultanée d'artemisinine et d'huile essentielle à partir de la plante artemisia annua

(43) Date of publication of application: 14.04.1999
(73) Proprietor: Council of Scientific and Industrial Research, New Delhi 110 001 (IN)
(72) Inventor: Jain, Dharam Chand, P.O CIMAP, Lucknow 226015 (IN); Tandon, Sudeep, P.O CIMAP, Lucknow 226015 (IN); Bhakuni, Rajendra Singh, P.O CIMAP, Lucknow 226015 (IN); Siddique, Mohammed Shafique, P.O CIMAP, Lucknow 226015 (IN); Kahol, Atul Prakash, P.O CIMAP, Lucknow 226015 (IN); Sharma, Ram Prakash, P.O CIMAP, Lucknow 226015 (IN); Kumar, Sushil, P.O CIMAP, Lucknow 226015 (IN); Bhattacharya, Asish Kumar, P.O CIMAP, Lucknow 226015 (IN)
(74) Representative: Portal, Gérard

(56) References cited:
- US-A- 4 952 603

## Description

The present invention relates to process for the simultaneous production of essential oil and artemisinin from the plant *Artemisia annua.* More particularly. the invention is related to a process for the production of essential oil and artemisinin and conversion of artemisinic acid into artemisinin.

*Artemisia annua* L. (Asteraceae) is a herb of Asiatic and Eastern European origin that has been also naturalised in USA. This species is receiving considerable attention because of the antimalarial activity of artemisinin of formula (1) shown in the accompanying drawings, a sesquiterpene lactone endoperoxide which is present in its aerial parts. In clinical trials, mainly performed in southeast Asia, artemisinin and a series of semi-synthetic derivatives have been proved to be effective against Plasmodium parasites with resistance to the commonly used antimalarial drugs. Now, efforts are being made to make these drugs cheaply available worldwide. In addition, *A. annua* is valued for its essential oil which has characteristic sweet aroma. Its application in perfumery, cosmetics, aromatherapy and as an antimicrobial, dermatological, fungicidal agents may provide an additional market for essential oil.

Artemisinic acid of formula (2) shown in the accompanying drawings is the biosynthetic precursor of artemisinin, which are generally present in greater quantity than artemisinin in *A. annua* plant. Artemisinic acid can be converted into artemisinin in good yield, thereby artemisinin amount obtained directly from the plant is increased 3-4 folds.

### PRIOR ART REFERENCES OF THE INVENTION

So far, no prior art literature has provided a method for the simultaneous isolation of artemisinin and essential oil from *A. annua* plant without destroying any of them. At present, the herb *A. annua* is processed for obtaining the essential oil by hydrodistillation method (Woerdenbag, H.J.; Pras, N; Chen, N.G., Bang Bui-Thi; Bos, R., Uden Wim Van; Pham Van Y; Boi, N.V; Batterman, S. and Lugt. C. (1994), Artemisinin related sesquiterpenes and essential oil in *Artemisia annua*, during vegetation period in Vietnam, Planta Medica, 60, 272-75). In this process, the fresh herb and water are heated in Clevenger apparatus at 100°C for 3-4 hrs. The steam distillate is condensed and essential oil is collected but in this process artemisinin gets destroyed. As artemisinin is a thermally labile compound, it gets decomposed during hydrodistillation.

In another process, to isolate artemisinin and other biogentic precursors, the dried plant material is extracted with non polar solvent (hexane), partitioning the hexane extract between hexane and acetonitrile, followed by chromatographying the acetonitrile phase over silica gel, elution with solvent afforded different fractions, which on concentration and crystallization yielded artemisinin, artemisinic acid, arteannuin B, but no essential oil is recovered in this process. Klayman, D.L.; Lin, A.J. Acton, N; Scovill, J.P.; Hoch, J.M.; Michous, W.K.; Theoharidis, A.D. and Dobek, A.S. J. Nat. Prods. 47, (1984) 715. Another disadvantage in the above process is that the artemisinic acid being predominant, it tends to elute with artemisinin, thus affecting the purity of the desired compound.

An improved method for the isolation of artemisinin acid from *A.annua* plant (Vonwiller, S.C, Hayne, R.K, King, G. and Wang, II. Planta medica 59, 562-563 (1993) was reported. In this process, methanolic extract of *A.annua* were partitioned between water and ether solvent. Ether residue was treated with base to separate artemisinic acid fraction from artemisinin. The basic solution was neutralized and further methylation of residue stirring with acid. After methylation the same base extraction process was repeated to obtain artemisinic acid. The residue left after base extraction was chromatographed to obtain artemisinin which afforded 57 % of yield. In this process, total extract was treated with base which destroyed some amount of artemisinin. The solvent namely ether used in the process is low boiling and highly inflammable.

Artemisinic acid is the most abundant metabolite of *A.annua* and its conversion to artemisinin would increase the yield of artemisinin. Roth. R.J and Acton. N. J.Chem.Edu. 68,612 (1991) have prepared dihydroartemisinic acid by using excess quantity of NaBH₄ and NiCl₂.6H₂O in methanol. Dihydroartemisinic acid was photo-oxidised at (-) 78°C in dichloro methane or at 0°C in solvent acetone with methylene blue as photosynthesiser and oxygen was passed through the solution with irradiating with high intensity lamp. The solution was evaporated and the residue was taken up in ether and filtered the solution to remove dye. The solvent was evaporated, residue was re-dissolved in pet. ether, containing a few drops of trifluoroacetic acid and the photolysate was left for four days to afford 17-30% artemisinin. In this process, photo oxidation was carried out at low temperature and also used number of solvents, chemical and process steps.

US patent 4,952,603 teaches a method for the isolation of artemisinin from the plant Artemisia annua; said method comprises the steps of extracting the leaves of the plant with hexane, partitioning the hexane between hexane and acetonitrile following chromatographing the acetonitrile phase to produce substantially pure artemisinin. This method teaches production of artemisinin and involves chromatography whereby artemisinic acid is eluted predominantly. The essential oil from the plant Artemisia annua cannot be extracted by this process.

### SUMMARY OF THE INVENTION

The present invention provides a simple and efficient process for the simultaneous production of artemisinin and essential oil from the plant *A. annua* and also a method for better recovery of artemisinic acid and artemisinin without the use of chromatography and finally conversion of artemisinic acid into artemisinin.

### NOVELTY OF THE INVENTION

1. In the prior art, essential oil is obtained by hydro-distillation of fresh/dried plant material in which artemisinin gets destroyed and the essential oil is obtained after 3 hrs. contact with steam, whereas in this present invention, the dried plant material is extracted with n-hexane and the extract is partitioned with aqueous acetonitrile. In this step, fatty material is seperated from artemisinin, so that purification and recovery of artemisinin is improved as well as essential oil is obtained by hydrodistillation of marc and hexane residue obtained in the partition step in which no artemisinin is present. Therefore, in this process, no loss of artemisinin takes place. For the first time, 50% essential oil is obtained from the marc of the plant. The quality of the oil is better and less time is required for hydrodistillation. For the first time, the applicants have achieved the simultaneous production of artemisinin and essential oil without destroying either of them.
2. In the previous process artemisinic acid was seperated from aqueous acetonitrile phase by the treatment with sodiumcarbonate or chromatography. In this process, artemisinin present also gets decomposed and only 57% artemisinin was recovered. But in the present invention, artemisinic acid will be seperated from artemisinin before treatment with base, so that both the compounds are recovered in 90% yield.
3. The conversion of artemisinic acid (obtained from *A.annua*) into artemisinin results in the best utilization of the compounds obtained during the process as well as increases the yield of artemisinin from the plant. As the synthesis of artemisinin is not economically viable plant remains the sole source for its large scale production. In the present improved process, conversion takes place in only two steps instead of the three steps as used in the prior art. The present process does not use any catalyst, photosynthesiser and oxygen. The reaction takes place at room temperature and work up of the reaction is very simple to obtain artemisinin.

### SUMMARY OF THE INVENTION

According to a first aspect, the present invention relates to a process for the simultaneous production of essential oil and artemisinic acid optionally converted into artemisinin, from the plant *Artemisia annua*, comprising the following steps:
a) extracting said plant with a non polar organic solvent, thereby obtaining a non polar organic extract;
b) partitioning the non polar organic extract between an organic phase and an aqueous phase;
c) evaporating the organic phase substantially to dryness thereby obtaining an organic phase residue, and performing a hydro-distillation of the residue preferably admixed with the plant marc yielding said essential oil
d) purifying the aqueous phase to yield artemisinic acid; and
e) optionally converting artemisinic acid into artemisinin.

According to a second aspect, the present invention relates to a process which consists of the following steps:
i) drying and powdering *A. annua* plant,
ii) extracting said powdered *A. annua* plant with hexane,
iii) reducing the hexane extract obtained from step ii) above to 5-20% of its original volume under vacuum;
iv) partitioning the hexane extract between hexane and acetonitrile water mixture;
v) evaporation of the hexane phase obtained in step iv) to dryness,
vi) hydro-distillation of the hexane residue obtained from step v) and the marc (extracted plant material) to yield essential oil,
vii) removal of water from the aqueous acetonitrile phase obtained in step iv),
viii) further fractionation of the resultant acetonitrile phase obtained in step vii) between a hexane-benzene mixture to obtain hexane-benzene phase and acetonitrile phase,
ix) treating the hexane-benzene phase obtained in step viii) with a base followed by neutralisation, extraction with chloroform, drying and crystallisation to obtain artemisinic acid,
x) converting artemisinic acid obtained in step ix) into artemisinin by reduction and photo -oxidation,
xi) chromatographing the evaporated acetonitrile residue obtained from step viii) over silica gel with hexane, thereby providing fractions containing artemisinin, and
xii) evaporating the different fractions obtained from step xi) and crystallisation of said fractions containing artemisinin obtained from step xi) and thereby producing substantially pure artemisinin.

According to a third aspect, the present invention relates to the use of artemisinin obtained from the process according to the invention as an antimalarial drug.

Advantageous embodiments of all these aspects of the present invention are set forth in the description that follows, together with the sub-claims.

### DETAILED DESCRIPTION OF THE INVENTION

In accordance with the method of the invention, employing n-hexane solvent for extraction of the plant in which complete extraction of artemisinin and other bio-precursors and 50% of essential oil. The step of partitioning separates the fatty material from other products containing artemisinin, whereas defatting of crude extract with n-hexane, alcohol etc., results in the loss of artemisinin and filteration of fat is a difficult operation which is avoided in this process. In the previous process, the hydrodistillation of plant material to obtain essential oil, due to high temperature artemisinin gets destroyed during operations but here the applicants have separated the artemisinin fraction from fatty material which on hydrodistillation yield essential oil. In this process, the remaining essential oil in plant which is not extracted during hexane extraction which is to be identified and recovered from the extracted plant material (Marc) by hydrodistillation. The hydrodistillation of marc and concentrated hexane residue took less time and size of distillation unit is drastically reduced which is required during fresh plant extraction. During drying, the structure changes of the cells of plant material favour the diffusion of the oil components out of them.

The partitioning step in liquid-liquid extraction done in Karr type column at particular flow rate and stirring, transfer the artemisinin and two major sesquiterpene, in the acetonitrile phase (polar solvent ) with concomitant reduction of in the amount of extract, i.e. of the order of 20 - 24 % of the original hexane extract in the single operation.

The acetonitrile phase rich in artemisinic acid only 10% of the acid isolated by crystallization before chromatography. Here in this process, complete extraction of artmisinic acid by partitioning between acetonitrile and hexane-benzene mixture, which reduced the bulkness of the acetonitrile extract further by 50%. Using the method of the present invention, the second partitioning step which remove artemisinic acid prior to elution of artemisinin without chromatography, enhancing the purification of artemisinin, less consumption of silica gel, time and solvent and economize the cost of production of the drug-artemisinin. In the Vonwiller process, artemisinic acid was isolated from the ether extract by treatment with a base. In the ether extract, artemisinin which was also present gel decomposed during the base extraction. Here the applicants have separated the artemisinic acid fraction from artemisinin.

In the chromatography step of the invention, ratio of (1:3) (solute to adsorbent) was discovered to yield excellent results. In the known process, a ratio of 1:10 has been required. In the solvent system of the invention, n-hexane was found to be quite effective rather than 10-20% ethyl acetate in n-hexane mixture. The silica gel used as a packing material in the example here is silica gel-H approximate micrometer size 74 (Mesh size - 200). The elution of compounds done under reduced pressure. In this way elution of artemisinin by n-hexane rather than 15% ethyl acetate-hexane mixture, arteannuin B was recovered by 5% ethyl acetate hexane mixture. By way of chromatography step of the process of elution, artemisinin was obtained from the oily greenish yellow fraction eluted with hexane and purification of artemisinin was carried by crystallization from ethyl acetate-hexane (1:4). In accordance with the description herein used, solvent, silica gel and time for chromatography reduced drastically.

Accordingly, the present invention provides a novel process for the simultaneous production of essential oil and artemisinin from the *Artemisia annua,* said process comprising (i) drying and powedering *A. annua* plant ; (ii) extracting the said powdered herb of *A. annua* with hexane ; (iii) reducing the hexane extract obtained from step (ii) above to 5-20% of its original volume under vacuum ; (iv) partitioning the hexane extract between hexane and acetonitrile water mixture ; (v) evaporation of the hexane phase obtained in step (iv) to dryness : (vi) hydrodistillation of hexane residue obtained from the step (v) and Marc (extracted plant material) to yield essential oil ; (vii) removal of water from aqueous acetonitrile phase obtained from step (iv); (viii) further fractionation of resultant acetonitrile phase after removal of water as obtained from step (vii) between hexane-benzene mixture to obtain hexane-benzene extract and acetonitrile phase ; (ix) treating hexane-benzene fraction obtained from step (viii) with a base followed by neutralization, extraction with chloroform, drying and crystallisation to obtain artemisinic acid ; (x) converting artemisinic acid obtained from step (ix) into artemisinin by reduction and photo oxidation ; (xi) chromatographying of the evaporating acetonitrile residue obtained from step (viii) over silica gel with hexane ; (xii) evaporation of the different fractions obtained from step(xi) and crystallization of the said fractions containing artemisinin obtained from step (xi) and thereby producing substantially pure artemisinin.

The dried parts of the plant used for the extraction can be selected from any part of the plant, preferably leaves, influroscence and small stems and the hexane extract of the *A.annua* as used in step (i) is reduced to 10 % of its original volume under vacuum. Preferably, the partitioning between hexane and aqueous acetonitrile phase is done in the ratio of 2:3 in liquid-liquid extraction column and the aqueous acetonitrile mixture used is in the ratio of 1:1 to 1:5.

In the present process, the single extraction step (ii) is carried out between two phases for 3 hrs. and the hydrodistillation of Marc (extracted plant material) in step (i) and residue obtained from (iii) yield essential oil upto 2.0 hrs.

Also, in the present process, further partitioning between acetonitrile and hexane-benzene mixture after removal of water is done to isolate artemisinic acid and 10-30 % benzene used in hexane solvent was used for the extraction of artemisinic acid.

Preferably, extraction of artemisinic acid is carried out where hexane-benzene mixture is evaporated, extracted with 5% of sodium carbonate solution and the basic solution is neutralized with 5% HCl solution, extracting with chloroform. drying the solvent and crystallization with ethyl acetate yielding artemisinic acid.

One embodiment of the invention provides a process for the conversion of artemisnic acid into artemisinin employing steps such as reduction and photo oxidation and the reduction is carried out by the following steps comprising (i) dissolving artemisinic acid and NiCl₂.6H₂O in dry methanol ; (ii) adding sodium borohydride to the resultant solution at 0°C over a 2 hr. period : (iii) neutralising the solution obtained in step (ii) with 5% aqueous HCl solution and (iv) isolating and crystallizing the dihydro artemisinic acid in ethyl acetate to obtain pure dihydro artemisinic acid. However, the step of photo oxidation is carried out by comprising (i) dissolving the dihydroartemisinic acid in the solvent dichloromethane - ethyl acetate ; (ii) oxidising the solution obtained in die step (i) in the presence of fluorescent light (40W) daily for two hours upto 8 days : (iii) concentrating the solution obtained in step (ii) and recrystallizing the residue in hexane to isolate artemisinin.

Preferably, in the present process, the chromatographic step is carried out in SiO₂column comprising a solute having adsorbent ratio 1:3. The elution with n-hexane solvent is being done under vacuum at 0.133 - 0.2 bar (100 - 150 mm Hg) absolute pressure and the chromatographic adsorbent used is Silica gel H with approximate micrometer size 74 (mesh size 200).

The process of invention is illustrated by the following examples which should not be construed to limit the scope of the present invention.

The following examples also illustrates the specific embodiments of the method of the invention.

### Example 1

Dried powdered herb of *A.annua* (40 kg) was percolated with hexane ( 60 - 80°C) ( 6 x 200 liter) in a soxhlet for 8 hrs. The extracted solvent was reduced to 20 liter. The non polar phase (hexane extract) was partitioned with aqueous acetonitrile phase (1:5) presaturated each other in the liquid-liquid extraction (Karr type) column. After 3 hrs. two phases were separated. The aqueous acetonitrile phase was back washed using 10% of its volume with presaturated hexane (2.0 litre). Evaporation of non polar phase provided (2.20kg) residue. The residue was boiled in Clevenger apparatus with 10 litre water for 1.5 hrs. which yielded the essential oil (80ml). The marc (extracted plant material) (40kg) was hydrodistilled in a distillation unit, which yield essential oil (100 ml) in 1 hr. The water from aqueous acetonitrile phase was removed by adding 1 kg sodium chloride. The acetonitrile solvent (25.0 litre) was further partitioned between acetonitrile extract and 10% benzene-hexane mixture in the same karr type column. After separation of two phases, evaporated the benzene-hexane mixture yielded (200gm) of residue. The residue (200gm) was dissolved in chloroform (400 ml) and extracted with 5% Na₂ CO₃, solution (3 x 500 ml). Basic solution was neutrilized with 10% HCI solution (400 ml). The neutral solution was extracted with chloroform, concentrated and crystallization with ehtylacetate yielded artmisinic acid (71.8 gm). The acetonitrile phase after concentration provide a residue (300 gm) which was filtered over silica gel (900 gm) under vacuum at 0.133 - 0.2 bar (100 - 150 mm Hg) absolute pressure. Artemisinin was obtained from the viscous greenish yellow fraction elutes with n-hexane. Purification of artemisinin was carried by recrystallization with ethyl acetate/hexane (1:4) to yield artemisinin (18 gm) Arteannuin B was obtained from the fraction eluted with 5% ethyl acetate in hexane. Evaporation of the fraction and crystallization yielded arteannuin B (36.5kg).

The above procedure was repeated two times on the same scale and consistently provided the same yield of artemisinin and other constituents.

### Example 2

The process followed as in example 1 in which the organic phase (20 litre) was partitioned with aqueous acetonitrile phase ( 1:5, 30 litre) in same extraction column for the same period of time. The acetonitrile phase after removal of water was dried over anhydrous sodiumsulphate and concentrated under reduced pressure to yield a residue (0.55 kg). This residue was chromatographed over silica gel (2.5 kg) and eluted with different ratio of ethyl acetate-hexane. Artemisinin was obtained from column fractions eluted with 8% ethyl acetate-hexane. Evaporation and crystallization with hexane ethyl acetate mixture afforded pure artemisinin (18.0 gm). fraction eluted from 5% ethyl acetate-hexane yielded artemisinic acid (70.6gm) where as 12% ethyl acetate in hexane fraction afforded arteannuin B (35.8 gm).

### Example 3

The process of extraction of *A.annua* was followed as in example 1 in which the hexane phase (20 litre) was partitioned with aqueous acetonitrile phase (1:3, 30 litre) in same extraction column for the same period of time and speed. After partitioning the hexane phase was concentrated to yield (1.82 kg)residue which on hydrodistillation yielded essential oil (50 ml). The acetonitrile extract was concentrated after removal of water and yielded viscous mass (0.8 kg). This viscous mass was chromatographed over silica gel ( 4.0 kg ) and eluted with different ratio cf solvent ethyl acetate-hexane. Artemisinin was obtained from column fractions eluted with 10% ethyl acetate-hexane. Evaporation and crystallisation with hexane ethyl acetate mixture afforded pure artemisinin (17.0 gm).

### Example 4

Dried herb of *A.annua* (40.0kg) was extracted with n-hexane in a soxhlet apparatus. The extracted solvent was reduced to 20 litre. The hexane soluble fraction (10 litre) was partitioned with aqueous acetonitrile phase (1:1, 30 litre) in same extraction column. The hexane soluble fraction obtained after partitioning was evaporated and residue (1.70 kg) was boiled in Clevenger apparatus with 8 liter water for 3 hrs. which yield the essential oil (40ml). The acetonitrile phase was concentrated and yielded viscous mass (1.24kg) which on chromatograph on silica gel as per example 3 yielded pure artemisinin (16.2gm).

### Example 5

Artemisinic acid is isolated from *A.annua* extract as in example I was used for conversion into artemisinin. Artemisinic acid (100 mg) was dissolved in 100 ml methanol containing 150 mg NiCl₂.6H₂O, 300 mg of NaBH₄ powder was added in small portions over 2 hr. period to a stirred and cooled solution. After the reaction was completed, excess reducing agent was destroyed by adding 20 ml of 5% aqueous HCl. The mixture was filtered to remove the insoluble impurities and then the aqueous methanolic solution was extracted with ether which was washed with water, dried and concentrated to afford 105 mg of crude dihydroartemisinic acid. Dihydroartemisinic acid (100 mg) was dissolved in dichloromethane-ethyl acetate (7:3, 20 ml), and the reaction mixture was left for 8 days at room temperature and irradiated with fluorescent light (tubelight) (40W) for a period of 2 hrs. per day. Solvent was removed under vacuum and the residue was reciystallized with n-hexane to afford artemisinin(25 %). The new process of production of essential oil and artemisinin, the subject matter of this patent, offered a number of advantages such as :
1. The process for the dual production of artemisinin and essential oil (80 %) from *A.annua* has been developed for the first time.
2. Consumption of silica gel has decreased due to reduction of charging material upto 10% of its original hexane extract and ratio of adsorbent to solute is (1:3).
3. The partitioning step between n-hexane and aqueous acetonitrile phase allowed the selective transfer of artemisinin and other sesquiterpenes into polar phase leaving non polar constituents in hexane solvent which results in a better method for the removal of large quantity of fat and other impurities, whereas removal of fats is difficult operation.
4. Hydrodistillation of extracted plant and hexane residue is a better alternative to recover major portion of the essential oil than hydrodistillation from fresh herb where artemisinin gets destroyed.
5. In this process, after recovery of essential oil from hexane residue, the applicants also obtained major fatty acids material which on further purification yield free fatty acids.
6. Isolation of artemisinic acid without chromatography, as by product, can be converted into artemisinin which will increase the overall yield of the artemisinin from the plant by 2-3 folds.
7. Conversion of artemisnic acid into atemisinin in two simple steps without using catalyst, dye, few solvents and oxygen. The present reaction takes place at room temperature.
8. The process is highly efficient and economical as most of the solvents and adsorbents used in the process are being recovered and reused.
9. All these advantages are significant economic value for large scale production of antimalarial drug artemisinin.

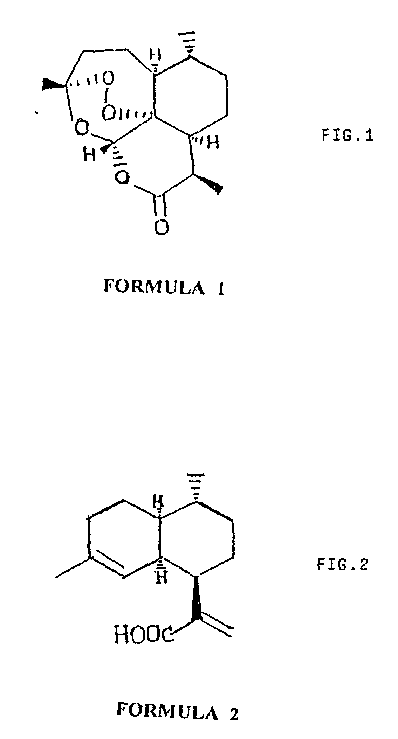

## Claims

1. A process for the simultaneous production of essential oil and artemisinic acid optionally converted into artemisinin, from the plant *Artemisia annua*, comprising the following steps:
a) extracting said plant with a non polar organic solvent, thereby obtaining a non polar organic extract;
b) partitioning the non polar organic extract between an organic phase and an aqueous phase;
c) evaporating the organic phase substantially to dryness thereby obtaining an organic phase residue, and performing a hydro-distillation of the residue preferably admixed with the plant marc yielding said essential oil
d) purifying the aqueous phase to yield artemisinic acid; and
e) optionally converting artemisinic acid into artemisinin.

2. The process of claim 1 wherein said non polar organic solvent comprises hexane.

3. The process according to claims 1 or 2, wherein said partitioning step of the non-polar organic extract is performed by adding an acetonitrile/water mixture to said non polar organic extract solvent which is preferably hexane.

4. The process according to any one of claims 1 to 3 wherein, prior to said partitioning step, said non polar organic extract is evaporated to 5-20% of its original volume, preferably under vacuum.

5. The process according to any one of claims 1 to 4, wherein said purification of the aqueous phase comprises the removal of water and further fractionation of resultant acetonitrile phase between a hexane-benzene mixture to obtain hexane-benzene phase and acetonitrile phase.

6. The process according to claim 5 wherein said hexane-benzene phase is neutralised with a base, extracted with chloroform thereby giving a chloroform phase, said chloroform phase is dried, and artemisinic acid is crystallised therefrom.

7. The process according to claim 6, wherein artemisinic acid is converted into artemisinin by reduction and photo-oxidation.

8. The process according to claim 5 wherein said acetonitrile phase is chromatographed over silica gel eluting with hexane and artemisinic acid or preferably artemisinin is recovered therefrom.

9. The process according to any one of claims 1 to 8, wherein said non polar organic solvent extraction of a) is performed on *A. annua* plant which has been dried and powdered.

10. A process for the simultaneous production of essential oil and artemisinin from the *Artemisia annua*, said process comprising:
i) drying and powdering *A. annua* plant,
ii) extracting said powdered *A*. *annua* plant with hexane,
iii) reducing the hexane extract obtained from step ii) above to 5-20% of its original volume under vacuum;
iv) partitioning the hexane extract between hexane and acetonitrile water mixture;
v) evaporation of the hexane phase obtained in step iv) to dryness,
vi) hydro-distillation of the hexane residue obtained from step v) and the marc (extracted plant material) to yield essential oil,
vii) removal of water from the aqueous acetonitrile phase obtained in step iv),
viii) further fractionation of the resultant acetonitrile phase obtained in step vii) between a hexane-benzene mixture to obtain hexane-benzene phase and acetonitrile phase,
ix) treating the hexane-benzene phase obtained in step viii) with a base followed by neutralisation, extraction with chloroform, drying and crystallisation to obtain artemisinic acid,
x) converting artemisinic acid obtained in step ix) into artemisinin by reduction and photo -oxidation,
xi) chromatographing the evaporated acetonitrile residue obtained from step viii) over silica gel with hexane, thereby providing fractions containing artemisinin, and
xii) evaporating the different fractions obtained from step xi) and crystallisation of said fractions containing artemisinin obtained from step xi) and thereby producing substantially pure artemisinin.

11. The process according to any one of claims 1 to 10 wherein said *Artemisia. annua* plant used for the extraction can be any part of the plant, preferably the leaves, inflorescence and small stems.

12. The process according to any one of claims 1 to 11 wherein the partitioning the non polar organic extract between said organic phase and said aqueous phase is done in the ratio of 2:3 in a liquid-liquid extraction column.

13. The process according to any one of claims 3 to 12 wherein said aqueous phase is an acetonitrile water mixture in the ratio of 1:1 to 1:5.

14. The process according to any one of claims 1 to 13 wherein partitioning step b) is carried out between said organic phase and said aqueous phase for 3 hours.

15. The process according to any one of claims 1 to 14 wherein said hydro-distillation of the residue is carried out for a period of time ranging from 30 minutes to 120 minutes.

16. The process according to any one of claims 5 to 15 wherein the benzene used in said hexane-benzene mixture used as solvent is used at a rate of between 10-30%.

17. The process according to any one of claims 6 to 16 wherein said base is 5% sodium carbonate solution.

18. The process according to any one of claims 7 to 17 wherein said reduction is carried out by the following steps comprising:
i) dissolving artemisinic acid and NiCl₂.6H₂O in dry methanol;
ii) adding sodium borohydride to the resultant solution at 0°C over a 2 hour period;
iii) neutralising the solution obtained in step ii) with 5% aqueous HCl solution, and
iv) isolating and crystallising the dihydro artemisinic acid in ethyl acetate to obtain pure dihydro artemisinic acid.

19. The process according to any one of claims 7 to 18 wherein said photo-oxidation comprises:
i) dissolving the dihydro artemisinic acid in a dichloromethane-ethyl acetate mixture;
ii) oxidising the solution obtained in the step i) in the presence of fluorescent light daily for two hours up to 8 days;
iii) concentrating the solution obtained in step ii) and recrystallising the residue in hexane to isolate artemisinin:

20. The process according to any one of claims 8 to 19 wherein said chromatographic step is carried out on a silica gel column which comprises a solute having an adsorbent ratio of 1:3.

21. The process according to any one of claims 8 to 20 wherein the elution with hexane solvent in said chromatographic step is done under vacuum of 0.133 - 0.2 bar (100-150 mm Hg) absolute pressure.

22. The process according to any one of claims 8 to 21 wherein the chromatographic adsorbent used in said chromatographic step is silica gel H with an approximate micrometer size of 74 (a mesh size of about 200).

## Patentansprüche

1. Verfahren zur gleichzeitigen Herstellung von ätherischem Öl und Artemisinsäure, die gegebenenfalls in Artemisinin umgewandelt ist, aus der Pflanze *Artemisia annua,* das die folgenden Schritte umfasst:
a) Extrahieren der Pflanze mit einem nicht polaren organischen Lösungsmittel, dadurch Erhalten eines nicht polaren organischen Extrakts;
b) Trennen des nicht polaren organischen Extrakts in eine organische Phase und eine wässrige Phase;
c) Verdampfen der organischen Phase bis zur wesentlichen Trockenheit, dadurch Erhalten eines organischen Phaserestes, und Durchführen einer Hydrodestillierung des Restes, der vorzugsweise mit dem Pflanzentrester vermischt ist, um das ätherische Öl zu ergeben
d) Reinigen der wässrigen Phase, um Artemisinsäure zu erhalten; und
e) gegebenenfalls Umwandeln von Artemisinsäure in Artemisinin.

2. Verfahren gemäß Anspruch 1, in dem das nicht polare organische Lösungsmittel Hexan umfasst.

3. Verfahren gemäß Anspruch 1 oder 2, wobei der Trennungsschritt des nicht polaren organischen Extrakts durch Hinzugabe einer Acetonitril/Wassermischung zu dem nicht polaren organischen Extraktionslösungsmittel, das bevorzugt Hexan ist, durchgeführt wird.

4. Verfahren gemäß einem der Ansprüche 1 bis 3, wobei der nicht polare organische Extrakt vor dem Trennungsschritt bevorzugt unter Vakuum auf 5-20% seines Ursprungsvolumens verdampft wird.

5. Verfahren gemäß einem der Ansprüche 1 bis 4, wobei die Reinigung der wässrigen Phase die Entfernung von Wasser und die weitere Fraktionierung der erhaltenen Acetonitrilphase in eine Hexan-Benzolmischung umfasst, um eine Hexan-Benzolphase und eine Acetonitrilphase zu erhalten.

6. Verfahren gemäß Anspruch 5, wobei die Hexan-Benzolphase mit einer Base neutralisiert wird, mit Chloroform extrahiert wird, dadurch eine Chloroformphase ergebend, wobei die Chloroformphase getrocknet wird und die Artemisinsäure hieraus kristallisiert wird.

7. Verfahren gemäß Anspruch 6, wobei Artemisinsäure durch Reduktion und Photooxidierung in Artemisinin umgewandelt wird.

8. Verfahren gemäß Anspruch 5, wobei die Acetonitrilphase durch ein Silikatgel chromatographiert, mit Hexan eluiert wird und daraus Artemisinsäure oder bevorzugt Artemisinin gewonnen wird.

9. Verfahren gemäß einem der Ansprüche 1 bis 8, wobei die nicht polare organische Lösungsmittelextraktion in a) mit einer A. annua Pflanze durchgeführt wird, die getrocknet und pulverisiert worden ist.

10. Verfahren zur gleichzeitigen Herstellung eines ätherischen Öls und Artemisinins aus *Artemisia annua,* wobei das Verfahren umfasst:
i) Trocknen und Pulverisieren der *A. annua* Pflanze,
ii) Extrahieren der pulverisierten *A. annua* Pflanze mit Hexan,
iii) Reduzieren des im obengenannten Schritt ii) erhaltenen Hexanextrakts unter Vakuum auf 5-20% seines Ursprungsvolumens;
iv) Trennen des Hexanextrakts in Hexan und eine Acetonitril-Wasser-Mischung;
v) Verdunsten der in Schritt iv) erhaltenen Hexanphase bis zur Trockenheit,
vi) Hydrodestillierung des in Schritt v) erhaltenen Hexanrests und des Tresters (extrahierten Pflanzenmaterials) um ätherisches Öl zu erhalten,
vii) Entfernen von Wasser aus der wässrigen Acetonitrilphase, die in Schritt iv) erhalten wurde,
viii)weitere Fraktionierung der in Schritt vii) erhaltenen Acetonitrilphase in eine Hexan-Benzolmischung, um eine Hexan-Benzolphase und eine Acetonitrilphase zu erhalten,
ix) Behandeln der in Schritt viii) erhaltenen Hexan-Benzolphase mit einer Base, gefolgt von einer Neutralisierung, Extrahierung mit Chloroform, Trocknen und Kristallisierung, um Artemisinsäure zu erhalten,
x) Umwandeln der in Schritt ix) erhaltenen Artemisinsäure in Artemisinin durch Reduktion und Photooxidierung,
xi) Chromatographieren des in Schritt viii) erhaltenen verdampften Acetonitrilrests durch ein Silikatgel mit Hexan, dadurch dem Erzeugen von Fraktionen, die Artemisinin enthalten, und
xii) Verdampfen der verschiedenen in Schritt xi) erhaltenen Fraktionen und Kristallisieren dieser Fraktionen, die Artemisinin enthalten, das in Schritt xi) erhalten wurde und dadurch dem Herstellen im wesentlichen reinen Artemisinins.

11. Verfahren gemäß einem der Ansprüche 1 bis 10, wobei die *Artemesia annua* Pflanze, die zur Extraktion verwendet wird, jeder Teil der Pflanze sein kann, bevorzugt die Blätter, Blütenstände und kleinen Triebe.

12. Verfahren gemäß einem der Ansprüche 1 bis 11, wobei das Trennen des nicht polaren organischen Extrakts zwischen einer organischen Phase und der wässrigen Phase in einem Verhältnis von 2:3 in einer Flüssig-Flüssig-Extraktionssäule durchgeführt wird.

13. Verfahren gemäß einem der Ansprüche 3 bis 12, wobei die wässrige Phase eine Acetonitril-Wasser-Mischung im Verhältnis von 1:1 bis 1:5 ist.

14. Verfahren gemäß einem der Ansprüche 1 bis 13, wobei das Trennen zwischen der organischen Phase und der wässrigen Phase in Schritt b) für 3 Stunden durchgeführt wird.

15. Verfahren gemäß einem der Ansprüche 1 bis 14, wobei die Hydrodestillierung des Restes über einen Zeitraum von 30 Minuten bis 120 Minuten durchgeführt wird.

16. Verfahren gemäß einem der Ansprüche 5 bis 15, wobei das verwendete Benzol, das in der verwendeten Hexan-Benzolmischung als Lösungsmittel verwendet wird, in einer Menge von 10-30% vorliegt.

17. Verfahren gemäß einem der Ansprüche 6 bis 16, wobei die Base 5% Natriumcarbonatlösung ist.

18. Verfahren gemäß einem der Ansprüche 7 bis 17, wobei die Reduktion durch die folgenden Schritte durchgeführt wird, die umfassen:
i) Lösen der Artemisinsäure und NiCl₂·6H₂O in trockenem Methanol;
ii) Hinzugabe von Natriumborhydrid zur resultierenden Lösung bei 0°C über einen Zeitraum von 2 Stunden;
iii) Neutralisieren der Lösung, die in Schritt ii) erhalten wurde mit 5% wässriger HCl-Lösung, und
iv) Isolieren und Kristallisieren der Dihydroartemisinsäure in Ethylacetat, um reine Dihydroartemisinsäure zu erhalten.

19. Verfahren gemäß einem der Ansprüche 7 bis 18, wobei die Photooxidierung umfasst:
i) Lösen der Dihydroartemisinsäure in einer Dichlormethan-Ethylacetatmischung;
ii) Oxidieren der Lösung, die in Schritt i) erhalten wurde in Gegenwart von Fluoreszenzlicht, täglich für zwei Stunden bis zu 8 Tagen;
iii) Konzentrieren der in Schritt ii) erhaltenen Lösung und Rekristallisieren des Restes in Hexan, um Artemisinin zu isolieren.

20. Verfahren gemäß einem der Ansprüche 8 bis 19, wobei der chromatographische Schritt mit einer Silikatgelsäule durchgeführt wird, die einen gelösten Stoff mit einem Absorptionsverhältnis von 1:3 umfasst.

21. Verfahren gemäß einem der Ansprüche 8 bis 20, wobei die Eluierung mit dem Hexan-Lösungsmittel im Chromatographieschritt unter einem Vakuum von 0,133 bis 0,2 bar (100-150 mm Hg) absolutem Druck durchgeführt wird.

22. Verfahren gemäß einem der Ansprüche 8 bis 21, wobei das chromatographische Absorptionsmittel, das in dem chromatographischen Schritt verwendet wird, Silikatgel H mit einer ungefähren Mikrometergröße von 74 ist (eine Maschengröße von ungefähr 200).

## Revendications

1. Procédé pour la production simultanée d'une huile essentielle et de l'acide artémisinique éventuellement converti en artémisinine, à partir de la plante *Artemisia annua*, comprenant les étapes consistant à :
a) extraire ladite plante avec un solvant organique non-polaire, obtenant ainsi un extrait organique non-polaire ;
b) partager l'extrait organique non-polaire entre une phase organique et une phase aqueuse ;
c) évaporer la phase organique sensiblement à sec, obtenant ainsi un résidu de la phase organique et effectuer une distillation par entraînement à l'eau du résidu de préférence mélangé au marc de la plante, ce qui donne ladite huile essentielle ;
d) purifier la phase aqueuse pour obtenir l'acide artémisinique et
e) éventuellement convertir l'acide artémisinique en artémisinine.

2. Procédé de la revendication 1, dans lequel ledit solvant organique non-polaire comprend de l'hexane.

3. Procédé selon les revendications 1 ou 2, dans lequel ladite étape de partage de l'extrait organique non-polaire est effectuée en ajoutant un mélange acétonitrile/eau audit solvant de l'extrait organique non-polaire qui est de préférence de l'hexane.

4. Procédé selon l'une quelconque des revendications 1 à 3, dans lequel, avant ladite étape de partage, ledit extrait organique non-polaire est évaporé jusqu'à 5 à 20 % de son volume d'origine, de préférence sous vide.

5. Procédé selon l'une quelconque des revendications 1 à 4, dans lequel ladite purification de la phase aqueuse comprend l'élimination de l'eau et un fractionnement supplémentaire de la phase acétonitrile obtenue, avec un mélange hexane-benzène, pour obtenir une phase hexane-benzène et une phase acétonitrile.

6. Procédé selon la revendication 5, dans lequel ladite phase hexane-benzène est neutralisée par une base, extraite par du chloroforme, obtenant ainsi une phase chloroformique, ladite phase chloroformique est séchée et l'acide artémisinique est cristallisé à partir de celle-ci.

7. Procédé selon la revendication 6, dans lequel l'acide artémisinique est converti en artémisinine par réduction et photo-oxydation.

8. Procédé selon la revendication 5, dans lequel ladite phase acétonitrile est chromatographiée sur du gel de silice avec élution par l'hexane, et l'acide artémisinique ou, de préférence, l'artémisinine, en est récupéré.

9. Procédé selon l'une quelconque des revendications 1 à 8, dans lequel ladite extraction par un solvant organique non-polaire de a) est effectuée sur une plante *A. annua* ayant été séchée et pulvérisée.

10. Procédé pour la production simultanée d'une huile essentielle et de l'artémisinine, à partir de *Artemisia annua,* ledit procédé comprenant :
i) le séchage et pulvérisation de la plante *A. annua*,
ii) l'extraction de ladite plante *A. annua* en poudre avec de l'hexane,
iii) la réduction de volume de l'extrait dans l'hexane obtenu dans l'étape ii) ci-dessus jusqu'à 5 à 20 % de son volume d'origine sous vide,
iv) le partage de l'extrait dans l'hexane entre de l'hexane et un mélange acétonitrile-eau,
v) l'évaporation à sec de la phase hexane obtenue dans l'étape iv),
vi) la distillation par entraînement à l'eau du résidu de la phase hexane obtenu dans l'étape v) et du marc (matière végétale extraite) pour obtenir l'huile essentielle,
vii) l'élimination de l'eau de la phase eau-acétonitrile obtenue dans l'étape iv),
viii) le fractionnement supplémentaire de la phase acétonitrile obtenue dans l'étape vii) avec un mélange hexane-benzène pour obtenir une phase hexane-benzène et une phase acétonitrile,
ix) le traitement de la phase hexane-benzène obtenue dans l'étape viii) avec une base suivie d'une neutralisation, d'une extraction par le chloroforme, d'un séchage et d'une cristallisation pour obtenir l'acide artémisinique,
x) la convertion de l'acide artémisinique obtenu dans l'étape ix) en artémisinine par réduction et photo-oxydation,
xi) la chromatographie du résidu de la phase acétonitrile évaporée obtenue dans l'étape viii) sur du gel de silice avec de l'hexane, obtenant ainsi des fractions contenant l'artémisinine, et
xii) l'évaporation des différentes fractions obtenues dans l'étape xi) et la cristallisation desdites fractions contenant de l'artémisinine obtenues dans l'étape xi) pour ainsi produire de l'artémisinine sensiblement pure.

11. Procédé selon l'une quelconque des revendications 1 à 10, dans lequel la plante *Artemisia annua* utilisée pour l'extraction peut être une quelconque des parties de la plante, de préférence les feuilles, l'inflorescence et les petites tiges.

12. Procédé selon l'une quelconque des revendications 1 à 11, dans lequel le partage de l'extrait organique non-polaire entre ladite phase organique et ladite phase aqueuse est réalisé dans un rapport de 2:3 dans une colonne d'extraction liquide-liquide.

13. Procédé selon l'une quelconque des revendications 3 à 12, dans lequel ladite phase aqueuse est un mélange acétonitrile - eau dans le rapport de 1:1 à 1:5.

14. Procédé selon l'une quelconque des revendications 1 à 13, dans lequel l'étape de partage b) est effectuée entre ladite phase organique et ladite phase aqueuse pendant 3 heures.

15. Procédé selon l'une quelconque des revendications 1 à 14, dans lequel ladite distillation par entraînement à l'eau, du résidu est effectuée pendant une durée de 30 minutes à 120 minutes.

16. Procédé selon l'une quelconque des revendications 5 à 15, dans lequel le benzène utilisé dans ledit mélange hexane-benzène utilisé comme solvant est utilisé à un taux de 10 à 30 %.

17. Procédé selon l'une quelconque des revendications 6 à 16, dans lequel ladite base est une solution de carbonate de sodium à 5 %.

18. Procédé selon l'une quelconque des revendications 7 à 17, dans lequel ladite réduction est effectuée par les étapes suivantes consistant à :
i) dissoudre l'acide artémisinique et NiCl₂.6H₂O dans du méthanol sec ;
ii) ajouter du borohydrure de sodium à la solution obtenue à 0°C sur une période de deux heures,
iii) neutraliser la solution obtenue dans l'étape ii) avec une solution aqueuse de HCl à 5 % et
iv) isoler et cristalliser l'acide dihydroartémisinique dans de l'acétate d'éthyle pour obtenir de l'acide dihydroartémisinique pur.

19. Procédé selon l'une quelconque des revendications 7 à 18, dans lequel ladite photo-oxydation comprend les étapes consistant à :
i) dissoudre l'acide dihydroartémisinique dans un mélange dichlorométhane-acétate d'éthyle ;
ii) oxyder la solution obtenue dans l'étape i) en présence d'une lumière fluorescente deux heures par jour pendant jusqu'à 8 jouis ;
iii) concentrer la solution obtenue dans l'étape ii) et recristalliser le résidu dans l'hexane pour isoler l'artémisinine.

20. Procédé selon l'une quelconque des revendications 8 à 19, dans lequel ladite étape de chromatographie est réalisée sur une colonne de gel de silice comprenant un soluté ayant un rapport à l'adsorbant de 1:3.

21. Procédé selon l'une quelconque des revendications 8 à 20, dans lequel l'élution par le solvant hexane dans ladite étape de chromatographie est réalisée sous un vide de 0,133 à 0,2 bar (100 - 150 mm de Hg) de pression absolue.

22. Procédé selon l'une quelconque des revendications 8 à 21, dans lequel l'adsorbant chromatographique utilisé dans ladite étape de chromatographie est du gel de silice H ayant une dimension micrométrique approximative de 74 (dimension en mesh d'environ 200).
